# EUROPÄISCHE PATENTSCHRIFT

(11) **EP 0 963 554 B1**
(45) Veröffentlichungstag und Bekanntmachung des Hinweises auf die Patenterteilung: **23.10.2002**
(21) Anmeldenummer: 97901617.7
(22) Anmeldetag: 30.01.1997
(51) Int. Cl.: G01N 33/543

(54) **VERFAHREN ZUR IMMUNOLOGISCHEN BESTIMMUNG EINES ANALYTEN**
METHOD FOR THE IMMUNOLOGICAL DETERMINATION OF AN ANALYTE
PROCEDE DE DETECTION IMMUNOLOGIQUE D'UN ANALYTE

(43) Veröffentlichungstag der Anmeldung: 15.12.1999
(73) Patentinhaber: MERCK PATENT GmbH, 64293 Darmstadt (DE)
(72) Erfinder: DREMEL, Bernd, D-64293 Darmstadt (DE)
(86) Internationale Anmeldenummer: EP9700403
(87) Internationale Veröffentlichungsnummer: WO98034114

(56) Entgegenhaltungen:
- EP-A- 0 170 446
- EP-A- 0 209 490
- EP-A- 0 297 290
- WO-A-86/05815
- DE-A- 19 547 346

## Beschreibung

Die Erfindung betrifft ein Verfahren zur immunologischen Bestimmung eines Analyten in einer Probelösung mit Hilfe von magnetischen und nicht-magnetischen Partikeln.

Immunologische Methoden zur Bestimmung von Analyten mit magnetischen und nicht-magnetischen Partikeln sind z.B. aus WO 95/04279 bekannt. Dabei werden nach der Inkubation die magnetischen Partikel durch ein magnetisches Feld in der Reaktionslösung niedergeschlagen; im Überstand wird dann der Analyt photometrisch bestimmt. In JP 05-52849 ist ein chromatographisches System beschrieben, das mit einem Magneten versehen ist. Das kapillare Medium erfüllt dabei die Aufgabe eines Filters. Die treibende Kraft, die den Analyten durch das Filter bzw. die Kapillare zwingt, ist die magnetische Kraft.

Die bekannten Verfahren haben eine Reihe von Nachteilen. Die magnetische Trennung der Partikel durch Niederschlagung in der Reaktionslösung hat den Nachteil, daß ein direkter photometrischer, fluorimetrischer oder elektrochemischer Nachweis des Analyten nicht ohne weitere Wasch- oder Trennschritte möglich ist. Ein Mangel der Filtrationssysteme besteht darin, daß die Filter bzw. Kapillaren leicht verstopien. Viskositätsprobleme auftreten und der Analyt vorzeitig auf dem chromatographischen Material, d.h. vor der Bindungsreaktion, adsorbiert wird, so daß nur ausgewähltes Probengut zuverlässig analysiert werden kann.

Der vorliegenden Erfindung liegt die Aufgabe zugrunde, die geschilderten Nachteile bei immunologischen Verfahren, die mit magnetischen Partikeln arbeiten, zu vermeiden und ein ganz einfaches und zuverlässiges Verfahren zur Verfügung zu stellen.

Gegenstand der vorliegenden Erfindung ist ein Verfahren zur immunologischen Bestimmung eines Analyten in einer Probe mit Hilfe von mit dem zu bestimmenden Analyten oder analytspezifischen Bindungspartnern beschichteten magnetischen Partikeln und mit analytspezifischen Bindungspartnern oder dem zu bestimmenden Analyten beschichteten, direkt nachweisbaren nicht-magnetischen Partikeln oder einer indirekt nachweisbaren nicht-magnetischen Substanz und Inkubation des Reaktionsgemisches, das dadurch gekennzeichnet ist, daß die magnetischen Partikel anschließend mit einem magnetischen Teststreifen aus dem Reaktionsgemisch abgetrennt und die Analytkonzentration direkt bestimmt wird.

Vorzugsweise wird die Analytkonzentration direkt auf dem magnetischen Teststreifen visuell oder reflektrometrisch bestimmt. Die Bestimmung kann auch indirekt nach Entwickeln einer Farbreaktion oder elektrochemisch auf dem Teststreifen erfolgen.

Unter Analyt werden vor allem die diagnostisch relevanten Inhaltsstoffe von Körperflüssigkeiten, d.h. Haptene oder Antigene verstanden; unter den analytspezifischen Bindungspartnern sind monoklonale und polyklonale Antikörper zu verstehen.

Die magnetischen Teststreifen nach der Erfindung können z.B. ähnlich wie Disketten, Ton- oder Videobänder aus geeigneten magnetisierbaren Pigmenten hergestellt werden. Vorzugsweise eignen sich sehr flache Permanentmagnete. Die Verwendung von Seltene-Erden-Metallegierungen erlaubt die Herstellung von Permanentmagneten mit besonders hoher Remanenz. Im einfachsten Fall kann für das erfindungsgemäße Verfahren ein flacher Dauermagnet verwendet werden, der vollständig mit weißem, glatten Karton umhüllt und mit Kunststoffklebstreifen an den Rändern verklebt wird. Der magnetische Teststreifen kann durch Abwischen der magnetischen Partikel von der glatten Oberfläche nach der Testdurchführung mehrfach verwendet werden.

Ein großer Vorteil der Magnetstreifentechnologie im Vergleich zur direkten klassischen Kopplung von Antikörpern an die Oberfläche von Teststreifen ist darin zu sehen, daß die Reaktion mit dem Analyten zunächst homogen bzw. quasi-homogen abläuft. Diffusionsprobleme, die den unteren Detektionsbereich bei heterogenen Reaktionen zwischen Teststreifen und Analyten einschränken, spielen im homogenen Milieu praktisch keine Rolle.

Umgekehrt werden bei heterogenen Reaktionen, die oft diffusionslimitiert sind, verschiedene Maßnahmen unternommen, den Stoffaustausch zwischen Reaktionsoberfläche und Analytlösung zu verbessern (Filtration des Analyten durch die Teststreifenoberfläche). Die mit diesen Maßnahmen erzielbaren Vorteile können jedoch nicht den Vorteil der quasihomogenen Reaktion zwischen Analyten und kleinen suspendierten Partikeln mit hoher Oberfläche kompensieren (Probleme infolge von Porendiffusion, Clogging-Effekte).

Als Partikel können im Prinzip alle im Handel erhältlichen magnetischen und nicht-magnetischen Partikel, die auch farbig sein können, verwendet werden, sofern sie eine Größe von 30 nm bis 800 µm haben. Geeignete Partikel sind anorganische und organische Partikel, z.B. magnetische Partikel aus Eisen, Nickel, Cobalt, Mangan, Elementen der Lanthanidenreihe wie Neodym, Erbium usw, Partikel aus magnetischen Legierungen wie Aluminium-, Nickel-, Cobalt-, Kupferlegierungen, Oxide wie Fe₃O₄ CrO₂, CoO, NiO₂, Mn₂O₃ usw., Kompositmaterialien wie Ferrite und organische Polymere wie Polystyrol.

Als erfindungsgemäße Verfahren ist vor allem zum Nachweis von Analyten geeignet, für die spezifischer Bindungspartner existiert. Die Durchführung erfolgt z.B. nach den bekannten Verfahren der Sandwich-Immunoassays oder der kompetitiven Immunoassays. Nach der Inkubation werden die magnetischen Partikel mit Hilfe des magnetischen Teststreifens abgetrennt, so daß die direkt nachweisbaren nicht-magnetischen Partikel bzw. die indirekt nachweisbare nicht-magnetische Substanz in der Reaktionsmischung zurückbleiben, sofern sie nicht an die magnetischen Partikel gebunden werden. Der Nachweis erfolgt auf der Oberfläche des Teststreifens entweder direkt optisch, z.B. durch die An- oder Abwesenheit andersfarbiger nicht-magnetischer Partikel, oder indirekt, z.B. wenn die nicht-magnetische Substanz ein Enzym ist, nach dem Entwickeln einer Farbreaktion auf dem Teststreifen. Handelt es sich bei der nicht-magnetischen Substanz um ein Enzym, so kann der Nachweis auf dem magnetischen Teststreifen auch elektrochemisch erfolgen. Bei dem Teststreifen handelt es sich dann um eine magnetische Elektrode.

Die direkte immunologische Bestimmung eines Analyten mit farbigen nicht-magnetischen Partikeln nach der Sandwich-Methode erfolgt z.B. so, daß ein Monoreagenz mit der Probelösung inkubiert wird. Das Monoreagenz besteht aus magnetischen Partikeln, die mit alalytspezifischen vorzugsweise monoklonalen Antikörpern beschichtet sind und andersfarbigen nicht-magnetischen Partikeln, die ebenfalls mit vorzugsweise monoklonalen Antikörpern beschichtet sind, wobei diese Antikörper gegen ein anderes Epitop des Analyten gerichtet sind. Die Partikel sind in einer geeigneten Pufferlösung suspendiert. Ist in der Probelösung der Analyt zugegen, so können die andersfarbigen nicht-magnetischen Partikel an die magnetischen Partikel binden. Nach Ablauf einer hinreichenden Reaktionszeit werden die magnetischen Partikel aus der Reaktionsmischung mit einem magnetischen Teststreifen abgetrennt. Die an die Oberfläche des Teststreifens gebundenen magnetischen Partikel werden direkt visuell mittels einer Farbskala oder mit einem Reflektometer ausgewertet. Die Farbänderung auf dem Teststreifen hängt direkt von der Anzahl der gebundenen nicht-magnetischen andersfarbigen Partikel ab und ist proportional zur Analytkonzentration. Zwei Wellenlängenmessungen können darüber hinaus Inhomogenitäten beim Aufziehen der magnetischen Partikel auf den Teststreifen ausgleichen.

Die immunologische Bestimmung nach der Methode des kompetitiven Assays wird z.B. mit einem Bireagenz durchgeführt. Reagenz 1 enthält in einer geeigneten Pufferlösung magnetische Partikel, die mit analytspezifischen (monoklonalen oder polyklonalen) Antikörpern beschichtet sind. Reagenz 2 enthält in der gleichen Pufferlösung andersfarbige nicht-magnetische Partikel, die mit dem zu bestimmenden Analyt beschichtet sind. Die Probelösung wird zunächst mit dem Reagenz 1 inkubiert. Ist in der Probelösung der Analyt vorhanden, so werden Bindungsstellen der analytspezifischen Antikörper auf den magnetischen Partikeln entsprechend der Konzentration des Analyten in der Probe abgesättigt. Nach einer geeigneten Inkubationszeit wird das Reagenz 2 zur Reaktionsmischung gegeben. Die mit dem Analyten beschichteten andersfarbigen nicht-magnetischen Partikel können an die magnetischen Partikeln binden, sofern nicht die Bindungsstellen der Antikörper durch den Analyten bereits blockiert sind. Nach Ablauf einer bestimmten Reaktionszeit werden die magnetischen Partikel aus der Reaktionsmischung mit dem erfindungsgemäßen magnetischen Teststreifen abgetrennt. Die an die Oberfläche des Teststreifens gebundenen magnetischen Partikel werden direkt visuell mittels einer Farbskala oder mit einem Reflektometer ausgewertet. Die Farbänderung auf dem Teststreifen hängt direkt von der Anzahl der gebundenen nicht-magnetischen andersfarbigen Partikel ab und ist umgekehrt proportional zur Analytkonzentration. Zwei Wellenlängenmessungen können darüber hinaus Inhomogenitäten bei dem Aufziehen der magnetischen Partikel auf den Teststreifen ausgleichen. Das Bireagenz kann auch so zusammengesetzt sein, daß die magnetischen Partikel mit dem Analyten beschichtet sind, und die nicht-magnetischen Partikel mit dem Antikörper; das Nachweisprinzip ist unverändert.

Eine indirekte immunologische Bestimmung eines Analyten mit einer nicht-magnetischen Substanz, z.B. einem Enzym, nach der Sandwich-Methode kann wie folgt durchgeführt werden. Ein Monoreagenz, das in einer Pufferlösung magnetische Partikel enthält, die mit einem analytspezifischen, vorzugsweise monoklonalen Antikörpern beschichtet sind, sowie eine nicht-magnetische Substanz, vorzugsweise ein Enzym, das mit vorzugsweise monoklonalen Antikörpern gekoppelt ist, jedoch einen Antikörper enthält, der gegen ein anderes Epitop des Analyten gerichtet als die Antikörper auf den magnetischen Partikeln. Dieses Monoreagenz wird mit der Probelösung inkubiert. Ist Analyt in der Probe vorhanden, so bindet die nicht-magnetischen Substanz an die magnetischen Partikel. Nach Ablauf einer bestimmten Reaktionszeit werden die magnetischen Partikel aus der Reaktionsmischung mit einem magnetischen Teststreifen abgetrennt. Anschließend wird auf dem Teststreifen eine chemische Reaktion zum Nachweis der nicht-magnetischen Substanz durchgeführt. Handelt es sich bei der nicht-magnetischen Substanz um ein Enzym, so kann beispielsweise ein auf dem Teststreifen fixierter Leukofarbstoff enzymatisch in einen Farbstoff umgewandelt werden. Die Farbänderung auf dem Teststreifen kann direkt visuell mittels einer Farbskala oder mit einem Reflektometer ausgewertet werden. Sie ist proportional zur Analytkonzentration.

Geeignete Pufferlösungen sind solche, die in der Lage sind, einen pH-Bereich von 5 bis 9 aufrechtzuerholen, wie Phosphatpuffer, Tris-, Borat-, HEPES-, PIPES-Puffer usw.

### Beispiel 1

### Bestimmung von Cortisol nach dem kompetitiven Assay

- Reagenz 1:: 50 mM Phosphatpufferlösung mit suspendierten magnetischen Partikeln, die mit anti-Cortisol-Antikörpern beschichtet sind.
- Reagenz 2:: 50 mM Phosphatpufferlösung mit an alkalische Phosphatase gekoppeltem Cortisol.

30 µl Probelösung (Serum) werden mit 150 µl Reagenz 1 3 Minuten vorinkubiert und anschließend weitere 3 Minuten mit 150 µl Reagenz 2 inkubiert. Durch kurzes Eintauchen des magnetischen Teststreifens in die Reaktionslösung (1 Minute) werden die suspendierten magnetischen Partikel an die Oberfläche des Teststreifens gebunden. Der Teststreifen wird anschließend für 1 Minute in die Substratlösung (para-Nitrophenylphosphatlösung) eingetaucht. Nach ca. 5 Minuten Reaktionszeit wird die Analytkonzentration qualitativ durch Farbvergleich mit einem Referenzteststreifen bestimmt.

Bei der Reaktion tritt ein Verdrängungswettbewerb zwischen dem gesamten Cortisol des Serums und dem mit alkalischer Phosphatasemarkierten Cortisol ein. Die Menge an markiertem Cortisol, das auf den Partikeln gebunden wird ist um so höher, je geringer die Menge an gesamtem Cortisol im Serum ist. Der Farbumschlag auf dem Teststreifen ist umgekehrt proportional zur Analytkonzentration.

### Beispiel 2

### Bestimmung von Ferritin

- Reagenz 1:: 0,2 M Tris-Pufferlösung mit suspendierten magnetischen Partikeln, die mit anti-Ferritin-Antikörpern beschichtet sind.
- Reagenz 2:: 0,2 M Tris-Pufferlösung mit an alkalische Phosphatase gekoppeltem Ferritin.

Die Bestimmung wird analog Beispiel 1 mit para-Nitrophenylphosphat als Substratlösung durchgeführt. Nach 4 Minuten Reaktionszeit wird die Ferritinkonzentration qualitativ durch Farbvergleich des magnetischen Teststreifens mit Referenzteststreifen bestimmt.

### Beispiel 3

### Bestimmung von TNT in Wasserproben

Das TNT-Reagenz besteht aus einer 50 mM Phosphatpufferlösung vom pH 7,4 mit suspendierten magnetischen Partikeln (100 µg/ml), die mit anti-TNT-Antikörpern (IgG1, Lot: 103726 von Fa. SDI) beschichtet sind (Reagenz 1), sowie einer gepufferten Kompetitionslösung (TNT-alkalische Phosphatase-Konjugat, Lot: 103881, Fa. SDI, 1:80 verdünnt in 50 mM TRIS-Puffer, pH 8,0, 0,15 M NaCl, 1 mM MgCl₂ · 6 H₂O, 0,1 mM ZnCl₂, 2,5 % BSA, 5 % Saccharose, 0.1 % NaN₃ (Reagenz 2).

100 µl Probe (TNT-Lösung in 40 mM HEPES-Puffer werden mit 150 µl Reagenz 1 2 Minuten vorinkubiert und anschließend weitere 2 Minuten mit 150 µl Reagenz 2 inkubiert. Durch Eintauchen des magnetischen Teststreifens in die Reaktionslösung (1 Minute) werden die suspendierten Magnetpartikel an die Oberfläche des Teststreifens gebunden. Der Teststreifen wird anschließend für 1 Minute in eine Substratlösung (para-Nitrophenylsulfatlösung) eingetaucht. Nach weiteren 4 Minuten Reaktionszeit wird die Analytkonzentration qualitativ durch Farbvergleich mit dem Referenzteststreifen bestimmt. Die Gesamtanalysezeit beträgt ca. 10 Minuten.

Bei der Reaktion tritt ein Verdrängungswettbewerb zwischen dem TNT der Wasserprobe und dem AP-markierten TNT ein. Die Menge an markiertem TNT, das auf den Partikeln gebunden wird, ist um so höher, je geringer der TNT-Gehalt der Wasserprobe ist. Nach der Reaktion werden die magnetischen Partikel auf dem Teststreifen gebunden und in der Substratlösung inkubiert. Der Farbumschlag auf dem Teststreifen ist umgekehrt proportional zur Analytkonzentration.

## Patentansprüche

1. Verfahren zur immunologischen Bestimmung eines Analyten in einer Probe mit Hilfe von mit dem zu bestimmenden Analyten oder analytspezifischen Bindungspartnern beschichteten magnetischen Partikeln und mit analytspezifischen Bindungspartnern oder dem zu bestimmenden Analyten beschichteten, direkt nachweisbaren nicht-magnetischen Partikeln oder einer indirekt nachweisbaren nicht-magnetischen Substanz und Inkubation des Reaktionsgemisches, **dadurch gekennzeichnet, daß** die magnetischen Partikel anschließend mit einem magnetischen Teststreifen aus dem Reaktionsgemisch abgetrennt und die Analytkonzentration direkt auf dem Testreifen bestimmt wird.

2. Verfahren zur immunologischen Bestimmung eines Analyten nach Anspruch 1, **dadurch gekennzeichnet, daß** die Analytkonzentration direkt auf dem magnetischen Teststreifen bestimmt wird.

3. Verfahren zur immunologischen Bestimmung eines Analyten nach den Ansprüchen 1 und 2, **dadurch gekennzeichnet, daß** die Analytkonzentration indirekt nach Entwickeln einer Farbreaktion oder elektrochemisch bestimmt wird.

## Claims

1. Procedure for the immunological determination of an analyte in a sample with the aid of magnetic particles coated with the analyte to be determined or analyte-specific binding components and directly detectable non-magnetic particles coated with analyte-specific binding components or the analyte to be determined, or of an indirectly detectable non-magnetic substance and incubation of the reaction mixture, **characterized in that** the magnetic particles are then removed from the reaction mixture using a magnetic test strip and the analyte concentration is determined directly on the test strip.

2. Procedure for the immunological determination of an analyte according to Claim 1, **characterized in that** the analyte concentration is determined directly on the magnetic test strip.

3. Procedure for the immunological determination of an analyte according to Claims 1 and 2, **characterized in that** the analyte concentration is determined indirectly by development of a colour reaction or electrochemically.

## Revendications

1. Procédé pour la détermination immunologique d'un analyte dans un échantillon au moyen de particules magnétiques revêtues de l'analyte à déterminer ou de partenaires de liaison spécifiques de l'analyte et de particules non-magnétiques revêtues de partenaires de liaison spécifiques de l'analyte ou de l'analyte à déterminer directement détectables, ou d'une substance non-magnétique indirectement détectable et incubation du mélange réactionnel, **caractérisé en ce que** les particules magnétiques sont ensuite séparées du mélange réactionnel avec une bande magnétique de test et **en ce que** la concentration en analyte est directement déterminée sur la bande de test.

2. Procédé pour la détermination immunologique d'un analyte selon la revendication 1, **caractérisé en ce que** la concentration en analyte est directement déterminée sur la bande magnétique de test.

3. Procédé pour la détermination immunologique d'un analyte selon les revendications 1 et 2, **caractérisé en ce que** la concentration en analyte est indirectement déterminée après développement d'une réaction colorée ou par voie électrochimique.
